(19) Europäisches Patentamt
European Patent Office
Office européen des brevets

(11) Veröffentlichungsnummer: **0 012 178**
**B1**

# (12) EUROPÄISCHE PATENTSCHRIFT

(45) Veröffentlichungstag der Patentschrift: 08.12.82

(51) Int. Cl.³: **C 07 D 263/56**, C 07 D 413/10, A 61 K 7/42, A 61 K 7/06

(21) Anmeldenummer 79104031.4

(22) Anmeldetag: 18.10.79

## (54) Benzoxazolderivate, Verfahren zu deren Herstellung, kosmetische Mittel enthaltend solche Verbindungen, Verwendung solcher Verbindungen als UV-A-Filter sowie Verfahren zum Schützen der Haut und Haare und Verfahren zum Stabilisieren kosmetischer Mittel.

(30) Priorität: 31.10.78 CH 11210/78
27.08.79 CH 7758/79

(43) Veröffentlichungstag der Anmeldung: 25.06.80 Patentblatt 80/13

(45) Bekanntmachung des Hinweises auf die Patenterteilung: 08.12.82 Patentblatt 82/49

(84) Benannte Vertragsstaaten: AT BE CH DE FR GB IT NL SE

(56) Entgegenhaltungen:
AT-A-143 756
AT-B-272 524
CH-A-472 220
DD-A-105 229
DE-A-2 201 968
DE-A-2 756 883
K. Venkataraman, The Chemistry of Synthetic Dyes, Vol. I (1952), S. 344 + 345
Fitzpatrick, Sunlight and Man (Tokyo 1974), S. 12

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Poncioni, Bruno, Dr., Unterwartweg 29, CH-4132 Muttenz (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 012 178 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Benzoxazolderivate, Verfahren zu deren Herstellung, kosmetische Mittel enthaltend solche Verbindungen, Verwendung solcher Verbindungen als UV-A-Filter sowie Verfahren zum Schützen der Haut und Haare und Verfahren zum Stabilisieren kosmetischer Mittel

Die Erfindung betrifft Benzoxazolderivate der allgemeinen Formel

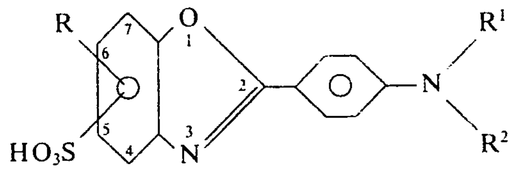

(I)

worin R Wasserstoff oder Alkyl mit 1 bis 4 Kohlenstoffatomen in 5- oder 6-Stellung und $R^1$ und $R^2$ unabhängig voneinander Wasserstoff, Alkyl mit 1 bis 4 Kohlenstoffatomen oder eine Gruppe $-(XO)_n-H$ bedeuten, worin X eine gegebenenfalls durch Methyl oder Äthyl substituierte Äthylengruppe mit 2 bis 4 Kohlenstoffatomen und n eine ganze Zahl von 1 bis 10 bedeuten, wobei mindestens eines der Symbole $R^1$ und $R^2$ von Wasserstoff verschieden ist, oder $R^1$ und $R^2$ zusammen mit dem Stickstoffatom, an das sie geknüpft sind, einen 5- bis 7gliedrigen, gesättigten Ring bilden, der gegebenenfalls noch ein weiteres Heteroatom enthält,
und Salze dieser Verbindungen.

Die Verbindungen der Formel I und deren Salze besitzen UV-A-absorbierende Eigenschaften und eignen sich insbesondere als UV-A-Filter für kosmetische Mittel.

Die schweizerische Patentschrift Nr. 472 220 offenbart bereits 2-Phenyl-benzoxazol-sulfonsäuren, die sich als UV-B-Absorber für kosmetische Mittel eignen. Die erfindungsgemäßen Verbindungen I unterscheiden sich jedoch von den bekannten dadurch, daß sie eine an die Phenylgruppe gebundene substituierte Aminogruppe aufweisen und ausgesprochene UV-A-Filter sind — und zwar solche UV-A-Filter, die den kritischen UV-A-Bereich genügend abdecken.

Ferner offenbart die österreichische Patentschrift Nr. 272 524 1,3-Bis-(5-sulfobenzimidazolyl-2)-benzol und dessen Salze als UV-B-Filter, von denen sich die erfindungsgemäßen Verbindungen I u. a. dadurch unterscheiden, daß sie eine Sulfobenzoxazolgruppe, nicht jedoch zwei Sulfobenzimidazolylgruppen, aufweisen und UV-A-Filter sind. Die in dieser Patentschrift als Stand der Technik erwähnte österreichische Patentschrift Nr. 143 756 erwähnt zwar u. a. 2-Phenyl-benzoxazol-sulfonsäure generisch, und zwar ebenfalls als UV-B-Filter. Ein Hinweis auf mit Amino substituierte Phenylreste findet sich hier nicht.

Die Erfindung betrifft ferner ein Verfahren zur Herstellung der Verbindungen der Formel I und deren Salze, kosmetische Mittel, die Verbindungen der Formel I und/oder deren Salze als UV-A-Filter enthalten, und ein Verfahren zu deren Herstellung sowie die Verwendung dieser Verbindungen.

Die Alkylgruppen können geradkettig oder verzweigt sein. Der Ausdruck »Alkyl mit 1 bis 4 Kohlenstoffatomen« umfaßt daher Methyl, Äthyl, n-Propyl, Isopropyl und n-, iso-, sec- und tert-Butyl.

Die Bedeutung »durch Methyl oder Äthyl substituierte Äthylengruppe« bezieht sich insbesondere auf

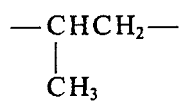 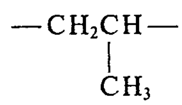 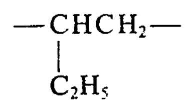 bzw. 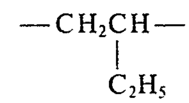

Falls mehrere Symbole X vorkommen, so sind sie unabhängig voneinander.

Die 5- bis 7gliedrigen, gesättigten Ringe können ein weiteres Heteroatom enthalten. Bevorzugte Heteroatome sind das Sauerstoff-, Stickstoff- und das Schwefelatom. Beispiele für solche Ringe mit und ohne Heteroatom sind Piperidino, Pyrrolidino, Morpholino, Piperazino und Thiomorpholino. Piperidino, Pyrrolidino, Morpholino und Thiomorpholino sind besonders bevorzugt.

Unter Salzen der Verbindungen der Formel I sollen die durch Neutralisieren der Verbindungen der Formel I mit einer entsprechenden Base erhältlichen verstanden werden, insbesondere die Alkalimetallsalze, wie beispielsweise die Natrium- und Kaliumsalze, die Ammoniumsalze und die Monoäthanolamin-, Diäthanolamin- und Triäthanolaminsalze. Besonders bevorzugte Salze sind die Triäthanolaminsalze.

Im Falle, daß R die Bedeutung Alkyl besitzt, so ist dies vorzugsweise Methyl.

Unabhängig voneinander bedeuten R vorzugsweise Wasserstoff, $R^1$ und $R^2$ vorzugsweise Methyl.

Die Verbindungen der Formel I, welche die $HO_3S$-Gruppe in 5- oder 6-Stellung, insbesondere in 5-Stellung, tragen, und deren Salze sind bevorzugt. Die einzelne Verbindung 2-(p-Dimethylaminophenyl)-benzoxazol-5-sulfonsäure und deren Salze sind besonders bevorzugt.

Eine interessante Untergruppe von Verbindungen der Formel I besteht aus den Verbindungen der allgemeinen Formel

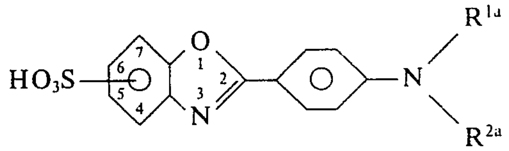

(Ia)

worin $R^{1a}$ und $R^{2a}$ unabhängig voneinander Wasserstoff, Alkyl mit 1 bis 4 Kohlenstoffatomen oder eine Gruppe $-X_aOH$ bedeuten, worin Xa eine gegebenenfalls mit Methyl oder Äthyl substituierte Äthylengruppe mit 2 bis 4 Kohlenstoffatomen bedeutet, wobei mindestens eines der Symbole $R^{1a}$ und $R^{2a}$ von Wasserstoff verschieden ist, oder $R^{1a}$ und $R^{2a}$ zusammen mit dem Stickstoffatom, an das sie geknüpft sind, einen 5- bis 7gliedrigen, gesättigten Ring bilden, der gegebenenfalls noch ein weiteres Heteroatom enthält,
und Salzen dieser Verbindungen.

Das erfindungsgemäße Verfahren zur Herstellung der Verbindungen der Formel I und von deren Salzen ist dadurch gekennzeichnet, daß man

a) eine Verbindung der Formel

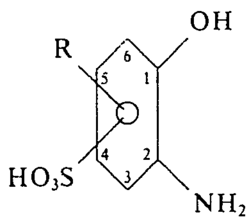

(II)

worin R Wasserstoff oder Alkyl mit 1 bis 4 Kohlenstoffatomen in 4- oder 5-Stellung bedeutet, mit einer Verbindung der Formel

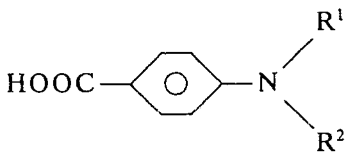

(III)

worin $R^{1'}$ Wasserstoff oder Alkyl mit 1 bis 4 Kohlenstoffatomen und $R^{2'}$ Alkyl mit 1 bis 4 Kohlenstoffatomen bedeuten oder $R^{1'}$ und $R^{2'}$ zusammen mit dem Stickstoffatom, an das sie geknüpft sind, einen 5- bis 7gliedrigen, gesättigten Ring bilden, der gegebenenfalls noch ein weiteres Heteroatom enthält,
oder einem reaktionsfähigen Derivat dieser Verbindung, in Gegenwart eines Kondensationsmittels umsetzt, oder

b) eine Verbindung der Formel I, worin mindestens eines der Symbole $R^1$ und $R^2$ Wasserstoff bedeutet und das andere, wenn nicht Wasserstoff, Alkyl ist, oder ein Salz einer solchen Verbindung, mit einem Alkylenoxid oder einem Gemisch von Alkylenoxiden mit 2 bis 4 Kohlenstoffatomen umsetzt, oder

c) eine Verbindung der Formel

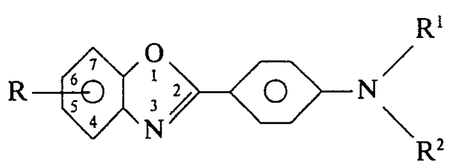

(IV)

worin R Wasserstoff oder Alkyl mit 1 bis 4 Kohlenstoffatomen in 5- oder 6-Stellung bedeutet, und $R^1$ und $R^2$ die vorstehend angegebene Bedeutung besitzen,

mit einem Sulfonierungsmittel behandelt,
und erwünschtenfalls eine erhaltene Verbindung der Formel I mit einer Base in ein Salz überführt.

Nach a) erfolgt die Herstellung von Verbindungen der Formel I beispielsweise durch Kondensation von 1 Äquivalent der Sulfonsäure der Formel II mit 1 Äquivalent des Benzoesäurederivats der Formel III oder eines reaktionsfähigen Derivats davon in Gegenwart eines Kondensationsmittels, wie beispielsweise Polyphosphorsäure. Die Reaktion wird vorzugsweise in einem Temperaturbereich zwischen 120 und 180°C durchgeführt. Die Kondensationsreaktion kann jedoch auch nach einer der anderen aus der Literatur bekannten Variationen dieses Verfahrens durchgeführt werden, wie

beispielsweise in dem Artikel von D. W. Hein et al. in J.A.C.S. 79, S. 427 (1967), beschrieben. So kann man z. B. die gewünschte Verbindung auch durch Kondensation einer Sulfonsäure der Formel II mit einem reaktionsfähigen Derivat einer Benzoesäure der Formel III, wie einem Ester, dem Amid, dem Chlorid oder dem Nitril, erhalten.

Die Reaktion zwischen einer Verbindung der Formel I, worin mindestens eines der Symbole $R^1$ und $R^2$ Wasserstoff bedeutet und das andere, wenn nicht Wasserstoff, Alkyl ist, oder einem Salz einer solchen Verbindung, und dem Alkylenoxid, insbesondere Äthylenoxid, Propylenoxid oder Butylenoxid, oder einem Gemisch mehrerer Alkylenoxide erfolgt nach b) zweckmäßigerweise in einem Molverhältnis Verbindung der Formel I : Alkylenoxid(e) von mindestens 1 : 1 bis 1 : 10 (wenn die Verbindung der Formel I eine Monoalkylaminogruppe aufweist) bzw. von mindestens 1 : 1 bis 1 : 20 (wenn die Verbindung der Formel I eine unsubstituierte Aminogruppe aufweist). Vorzugsweise erfolgt die Reaktion in der Abwesenheit eines Lösungsmittels und bei einer Temperatur zwischen etwa 60 und 120° C. Je nach Temperaturbereich und Ausgangsverbindungen dauert die Reaktion in der Regel zwischen etwa 2 und 6 Stunden.

Nach c) wird eine Verbindung der Formel IV mit einem Sulfonierungsmittel wie beispielsweise Oleum oder Chlorsulfonsäure behandelt. Diese Umsetzung erfolgt zweckmäßigerweise in der Abwesenheit eines Lösungsmittels und in einem Temperaturbereich zwischen etwa 100 und 160° C.

Die Salze der Verbindungen der Formel I werden durch Neutralisieren der Verbindung der Formel I mit einer entsprechenden Base erhalten. Geeignete Basen sind beispielsweise Natriumhydroxid, Kaliumhydroxid, Ammoniak und Mono-, Di- und Triäthanolamine.

Die Endprodukte können nach an sich bekannten Methoden isoliert und gereinigt werden.

Die Ausgangsmaterialien der Formel II und III sind entweder bekannt oder können nach an sich bekannten Methoden hergestellt werden.

Die Herstellung von Verbindungen der Formel I, worin $R^1$ und $R^2$ Wasserstoff bedeuten, und von Verbindungen der Formel IV, die in den Verfahrensvarianten b) bzw. c) als Ausgangsmaterialien verwendet werden, kann in analoger Weise zur Herstellung nach der Verfahrensvariante a) durchgeführt werden.

Es ist bekannt, daß die Haut, die nicht durch Kleidung geschützt ist, sehr viel rascher altert. Dieser unerwünschte Effekt ist der Strahlung des Wellenlängenbereiches 320 bis 400 nm, dem sogenannten UV-A-Bereich, zuzuschreiben. Bisher bekanntgewordene Filter, die für kosmetische Zwecke verwendet werden können, haben nun entweder diesen kritischen Wellenlängenbereich nicht genügend abgedeckt oder können infolge ihrer Löslichkeitseigenschaften nicht in fett- und alkoholfreie Kosmetika eingearbeitet werden, da sie nicht oder nicht genügend wasserlöslich sind.

Die Verbindungen der Formel I besitzen nun im Wellenlängenbereich von 320 bis 390 nm eine ausgezeichnete Filterwirkung (vgl. nachstehende Tabelle) und sind — insbesondere in Form ihrer Salze — in genügender Weise wasserlöslich. Die Verbindungen der Formel I ermöglichen somit die Herstellung von fett- und alkoholfreien Kosmetika, die zusätzlich einen Filter für das UV-A-Gebiet enthalten.

Vergleich der Lichtabsorption einer Verbindung der Formel I
mit einem bekannten Filter im UV-A-Gebiet

Nachstehend wird die prozentuale Absorption von UV-Licht im Bereich 320—400 nm einer 1 mg-% wäßrigen Lösung von 2-(p-Dimethylaminophenyl)-benzoxazol-5-sulfonsäure mit einer 1 mg-% wäßrigen Lösung von 2-Hydroxy-4-methoxy-benzophenon-5-sulfonsäure verglichen (Schichtdicke der Lösung: 1 cm).

| UV-Licht Wellenlänge | Absorbierter Lichtanteil durch | |
|---|---|---|
| | 2-(p-Dimethylamino-phenyl)-benzoxazol-5-sulfonsäure | 2-Hydroxy-4-methoxy-benzophenon-5-sulfonsäure |
| nm | % | % |
| 320 | 81 | 47 |
| 330 | 90 | 43 |
| 340 | 94 | 32 |
| 350 | 94 | 17 |
| 360 | 92 | 7 |
| 370 | 85 | 3 |
| 380 | 57 | 1 |
| 390 | 20 | 0 |
| 400 | 0 | 0 |

Aus den Absorptionswerten geht hervor, daß 2-(p-Dimethylaminophenyl)-benzoxazol-5-sulfonsäure zwischen 320 und 400 nm stärker absorbiert als die Vergleichssubstanz. Es geht auch hervor, daß in der Nähe des sichtbaren Lichtes (360–400 nm) die 2-(p-Dimethylaminophenyl)-benzoxazol-5-sulfonsäure noch stark absorbiert, während die Vergleichssubstanz keine Absorption mehr aufweist.

Die erfindungsgemäßen UV-A-Filter der Formel I und deren Salze besitzen auch die Eigenschaft, Farbstoffe, die auf UV-A-Strahlung empfindlich sind und die üblicherweise in Kosmetika vorhanden sind, vor Zerstörung zu bewahren.

Die erfindungsgemäßen kosmetischen Mittel enthalten als UV-A-Filter eine wirksame Menge von mindestens einer der erfindungsgemäßen Verbindungen. Vorzugsweise enthalten die Mittel mindestens ein Salz der Verbindungen der Formel I, wobei das Triäthanolaminsalz besonders bevorzugt ist.

Die Menge des im erfindungsgemäßen kosmetischen Mittel enthaltenen UV-Filters der Formel I kann je nach Anwendungsgebiet des Mittels in weiten Grenzen schwanken, jedoch liegt sie zweckmäßigerweise im Bereich von 0,01 bis 10 Gewichtsprozent, bezogen auf das gebrauchsfertige kosmetische Mittel.

In Crèmes und Sonnenmilch zum Schutz der Haut gegen UV-A-Strahlung (Lichtschutzmittel) sind die Verbindungen der Formel I und/oder deren Salze im allgemeinen in einer Konzentration von 0,5 bis 10 Gewichtsprozent, vorzugsweise von 2 bis 4 Gewichtsprozent, enthalten. In solchen Mitteln ist vorzugsweise auch ein UV-B-Filter vorhanden.

Um Farbstoffe, die in Kosmetika enthalten sind, vor UV-A-Strahlung zu schützen, werden solchen Mitteln, wie beispielsweise Shampoos oder Lotionen, die Verbindungen der Formel I und/oder deren Salze vorzugsweise in einer Konzentration von 0,01 bis 0,5 Gewichtsprozent zugesetzt. Es können sowohl eine oder mehrere freie Säuren der Formel I als auch ein oder mehrere Salze von solchen Verbindungen, vorzugsweise ein Triäthanolaminsalz, zugesetzt werden. In gleichen bevorzugten Konzentrationen werden die Verbindungen der Formel I sowie deren Salze auch weiteren Haarpflegemitteln zugesetzt, wie beispielsweise Haarwassern, Haarkuren, Haarfestigern und Haarfärbemitteln, da auch Haar unter dem Einfluß der UV-A-Strahlung leidet.

In den obigen kosmetischen Mitteln können aber auch andere UV-A-Filter sowie UV-B-Filter vorhanden sein.

Um die erfindungsgemäßen kosmetischen Mittel herzustellen, vermischt man auf in der Kosmetik übliche Weise mindestens eine der erfindungsgemäßen Verbindungen mit in der Kosmetik üblichen Wirkstoffen, Hilfsstoffen und Trägermaterialien.

Gemäß dem erfindungsgemäßen Verfahren zum Schützen der Haut bzw. der Haare vor Strahlen des UV-A-Gebietes appliziert man auf die Haut bzw. die Haare das erfindungsgemäße kosmetische Mittel. Das erfindungsgemäße Verfahren zum Bewahren eines im kosmetischen Mittel befindlichen Farbstoffes vor Zerstörung unter dem Einfluß von UV-A-Strahlen ist dadurch gekennzeichnet, daß man mindestens eine der erfindungsgemäßen Verbindungen ins kosmetische Mittel einarbeitet.

Die nachstehenden Beispiele illustrieren die Erfindung. Alle Temperaturen sind in Grad Celsius (° C) angegeben.

Beispiel 1

In einen mit Rührer versehenen Kolben gibt man äquimolare Mengen von p-Dimethylamino-benzoesäure und 2-Aminophenol-4-sulfonsäure. Anschließend wird Polyphosphorsäure beigefügt, bis eine rührbare Paste entsteht. Die Mischung wird danach unter Rühren auf 160° erhitzt und 1 Stunde bei dieser Temperatur gehalten. Danach läßt man die dunkelbraune Flüssigkeit bis 100° abkühlen und gießt sie unter Rühren in eine große Menge kaltes Wasser. Das Produkt fällt aus, wird abfiltriert, mit Wasser gründlich gewaschen und danach in Wasser suspendiert. Man gibt verdünnte Natronlauge zu dieser Suspension, bis eine beinahe klare Lösung entsteht (pH 7 – 7,5). Die Lösung wird filtriert und anschließend bis auf pH 3 – 3,5 angesäuert. Die 2-(p-Dimethylaminophenyl)-benzoxazol-5-sulfonsäure fällt aus, wird abfiltriert, mit Wasser gewaschen und im Wärmeschrank unter Vakuum getrocknet. Smp. über 300°.

Beispiel 2

In einen mit Rührer versehenen Kolben gibt man 17,5 g p-Dimethylamino-benzoesäure und 22,0 g 2-Aminophenol-4-sulfonsäure. Anschließend wird Polyphosphorsäure beigefügt, bis eine rührbare Paste entsteht. Die Mischung wird danach unter Rühren auf 160° erhitzt und 1 Stunde bei dieser Temperatur gehalten. Danach läßt man die dunkelbraune Flüssigkeit bis 100° abkühlen und gießt sie unter Rühren in eine große Menge eiskaltes Wasser. Das Produkt fällt aus, wird abfiltriert, mit Wasser gründlich gewaschen und danach in Wasser suspendiert. Man gibt eine verdünnte Ammoniaklösung zu dieser Suspension, bis eine beinahe klare Lösung entsteht (pH 7 – 7,5). Die dunkelbraune Lösung wird filtriert und anschließend bis auf pH 3 – 3,5 angesäuert. Die 2-(p-Dimethylaminophenyl)-benzoxazol-5-sulfonsäure fällt aus. Man filtriert ab, wäscht mit Wasser und trocknet im Wärmeschrank unter Vakuum. Fp. 338° (mit Zersetzung).

Analyse: $C_{15}H_{14}N_2O_4S$ (318,35)

| | | | | |
|---|---|---|---|---|
| berechnet: | C 56,59, | H 4,43, | N 8,80, | S 10,07%; |
| gefunden: | C 56,66, | H 4,53, | N 8,79, | S 9,96% ($H_2O$-frei). |

Beispiel 3

Man erhält auch 2-(p-Dimethylaminophenyl)-benzoxazol-5-sulfonsäure, wenn man 10,0 g p-Dimethylamino-benzonitril mit 13,0 g 2-Aminophenol-4-sulfonsäure in analoger Weise zum Beispiel 1 reagieren läßt.

Beispiel 4

Durch Umsetzung von 8,0 g p-Methylamino-benzoesäure mit 10,0 g 2-Aminophenol-4-sulfonsäure in analoger Weise zum Beispiel 1 erhält man 2-(p-Methylaminophenyl)-benzoxazol-5-sulfonsäure. Fp. 333° (mit Zersetzung).

Analyse: $C_{14}H_{12}N_2O_4S$ (304,32)

| | | | | |
|---|---|---|---|---|
| berechnet: | C 55,26, | H 3,97, | N 9,21, | S 10,53%; |
| gefunden: | C 54,90, | H 4,10, | N 9,14, | S 10,74% ($H_2O$-frei). |

Beispiel 5

Unter gleichen Bedingungen wie im Beispiel 1 läßt man 11,0 g p-Diäthylamino-benzoesäure und 10,5 g 2-Aminophenol-4-sulfonsäure reagieren. Nach der Reaktion wird die dunkelbraune Flüssigkeit in Wasser gegossen. Die Lösung wird mit Natronlauge auf pH 8 eingestellt. Die basische Lösung wird filtriert und mit Kochsalz gesättigt, bis eine dunkelbraune Masse entsteht. Diese Masse wird vom Wasser abgetrennt, wieder in Wasser gelöst und das Wasser mit Kochsalz gesättigt. Man erhält auf diese Weise das Natriumsalz von 2-(p-Diäthylaminophenyl)-benzoxazol-5-sulfonsäure als Pulver, das sich aus Alkohol – Wasser kristallisieren läßt. Fp. 318°

Analyse: $C_{17}H_{17}N_2O_4SNa$ (368,38)
berechnet: C 55,43, H 4,65, N 7,60, S 8,70%;
gefunden: C 55,47, H 4,86, N 7,69, S 8,40% ($H_2O$-frei).

Beispiel 6

Wenn man 5,0 g 4-Piperidino-benzoesäure-äthylester mit 4,2 g 2-Aminophenol-4-sulfonsäure in analoger Weise zum Beispiel 1 reagieren läßt, erhält man 2-(p-Piperidinophenyl)-benzoxazol-5-sulfonsäure. Fp. 351° (mit Zersetzung).

Analyse: $C_{18}H_{18}N_2O_4S$ (358,41)
berechnet: C 60,32, H 5,06, N 7,82, S 8,95%;
gefunden: C 60,32, H 5,09, N 7,88, S 8,90% ($H_2O$-frei).

Beispiel 7

Wenn man 10,0 g 4-Pyrrolidinobenzoesäure-äthylester mit 8,7 g 2-Aminophenol-4-sulfonsäure in analoger Weise zum Beispiel 1 reagieren läßt, erhält man 2-(p-Pyrrolidinophenyl)-benzoxazol-5-sulfonsäure. Fp. 310° (mit Zersetzung).

Analyse: $C_{17}H_{16}N_2O_4S$ (344,39)
berechnet: C 59,29, H 4,68, N 8,13, S 9,31%;
gefunden: C 60,28, H 4,76, N 8,16, S 9,00% ($H_2O$-frei).

Beispiel 8

10,0 g 2-(p-Aminophenyl)-benzoxazol-5-sulfonsäure (erhalten wie im Beispiel 1) werden in 40 g flüssigem Äthylenoxid suspendiert. Das Reaktionsgefäß wird unter Druck während 4 Stunden auf 100° erwärmt. Nach Kühlung wird das überschüssige Äthylenoxid entfernt und die dunkelbraune zähflüssige Masse in Feinsprit gelöst. Diese Lösung wird mit Aktivkohle behandelt. Man filtriert, dampft das Lösungsmittel ab und trocknet den entstandenen Rückstand unter Vakuum. So erhält man eine dunkelrote honigartige Masse. UV-Spektrum $\lambda_{FSP}^{max}$ =347 nm.
Das Produkt besteht wahrscheinlich aus einer Mischung von Verbindungen der allgemeinen Formel

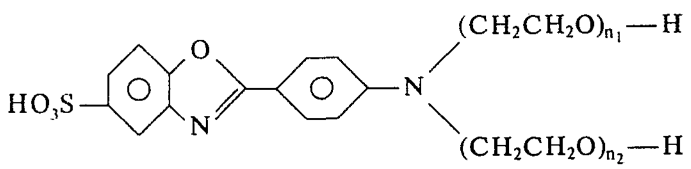

worin $n_1$ und $n_2$ unabhängig voneinander eine ganze Zahl von 1 bis 10 bedeuten.

Beispiel 9

11,2 g 2-(p-Dimethylaminophenyl)-benzoxazol werden in 26 ml konzentrierter Schwefelsäure gelöst. Danach gibt man 16 ml rauchende Schwefelsäure (Oleum 60%) zu. Man erwärmt 1 Stunde auf 200°. Nach dem Abkühlen wird die Lösung in 1200 ml Aceton gegossen. Die 2-(p-Dimethylaminophenyl)-benzoxazol-6-sulfonsäure fällt aus und wird nachher wie im Beispiel 1 verarbeitet. Fp. 315° (mit Zersetzung).

Analyse: $C_{15}H_{14}N_2O_4S$ (318,35)
berechnet: C 56,59, H 4,43, N 8,80, S 10,07%;
gefunden: C 56,20, H 4,33, N 8,85, S 10,19% ($H_2O$-frei).

Beispiel 10

Die analoge Reaktion wie im Beispiel 9 mit 2-(p-Dimethylaminophenyl)-5-methyl-benzoxazol führt zu 2-(p-Dimethylaminophenyl)-5-methyl-benzoxazol-6-sulfonsäure. Fp. 302,6° (mit Zersetzung).

Analyse: $C_{16}H_{16}N_2O_4S$ (332,37)

| | | | | |
|---|---|---|---|---|
| berechnet: | C 57,82, | H 4,85, | N 8,43, | S 9,65%; |
| gefunden: | C 57,33, | H 4,83, | N 8,70, | S 9,65% ($H_2O$-frei). |

## Beispiel 11

Die analoge Reaktion wie im Beispiel 9 mit 2-(p-Dimethylaminophenyl)-6-methyl-benzoxazol ergibt 2-(p-Dimethylaminophenyl)-6-methyl-benzoxazol-5-sulfonsäure. Fp. 290° (mit Zersetzung).

Analyse: $C_{16}H_{16}N_2O_4S$ (332,37)

| | | | | |
|---|---|---|---|---|
| berechnet: | C 57,82, | H 4,85, | N 8,43, | S 9,65%; |
| gefunden: | C 58,20, | H 4,95, | N 8,57, | S 9,42% ($H_2O$-frei). |

## Beispiel 12

Sonnenschutzmilch (Öl/Wasser-Emulsion)

| | |
|---|---|
| 5,000 g | Parsol®MCX (1) Äthylhexyl-paramethoxy-zimtsäureester |
| 3,000 g | Amphisol® (1) Diäthanolamincetylphosphat |
| 2,000 g | Stearylalkohol |
| 5,000 g | Cetiol®SN (2) Ester einer verzweigten Fettsäure mittlerer Kettenlänge mit Fettalkohol $C_{16}-C_{18}$ |
| 4,000 g | Paraffinöl dünnflüssig |
| 2,000 g | Butylstearat |
| 0,100 g | p-Hydroxybenzoesäure-propylester |
| 0,400 g | Carbopol®940 (3) Carboxyvinylpolymer |
| 5,000 g | 2-(p-Dimethylaminophenyl)benzoxazol-5-sulfonsäure |
| 0,630 g | Triäthanolamin |
| 0,700 g | Natriumhydroxyd |
| 2,000 g | Propylenglykol |
| 0,200 g | p-Hydroxybenzoesäure-methylester |
| 0,200 g | Germall®115 (4) Imidazolidinyl-harnstoffverbindung |
| 0,500 g | D-Panthenol-äthyläther |
| 0,500 g | D-Panthenol |
| 0,500 g | Parfum |
| ad 100 g | Entmineralisiertes Wasser |

## Beispiel 13

Halbfette Sonnenschutzcrème (Öl/Wasser-Emulsion)

| | |
|---|---|
| 5,000 g | Parsol®MCX (1) Äthylhexyl-paramethoxy-zimtsäureester |
| 5,600 g | Cremophor®A fest (5) Niedrig oxäthylierter gesättigter Fettalkohol |
| 5,000 g | Softisan®100 (6) Hartfettkomponente von ausschließlich gesättigten Pflanzenfettsäuren der Kettenlänge $C_{10}-C_{18}$ |
| 4,000 g | Cetylalkohol |
| 4,000 g | Stearylalkohol |
| 10,000 g | Sesamöl |
| 0,004 g | Butyl-hydroxy-anisol |
| 0,016 g | Butyl-hydroxy-toluol |
| 3,000 g | 2-(p-Dimethylaminophenyl)-benzoxazol-5-sulfonsäure |
| 0,360 g | Natriumhydroxyd |
| 0,200 g | Dowicil®200 (9) N-(3-Chlorallyl)-hexaminiumchlorid |
| 0,400 g | Parfum |
| ad 100 g | Entmineralisiertes Wasser |

Beispiel 14

Sonnenschutzlotion (Öl/Wasser-Emulsion)

| | |
|---|---|
| 5,000 g | Parsol®MCX (1) Äthylhexyl-paramethoxy-zimtsäureester |
| 5,000 g | Tagat®S (6) Polyoxyäthylen-glycerin-monostearat |
| 2,000 g | Glycerinmonostearat |
| 10,000 g | Deltyl®extra (1) Isopropylmyrisat |
| 8,000 g | Paraffinöl dünnflüssig |
| 2,000 g | Vaselin® weiß |
| 2,000 g | PCL flüssig (10) Gemisch alkylverzweigter Fettsäureester |
| 3,000 g | Stearylalkohol |
| 2,000 g | Miglyol®818 (8) Triglyceridgemisch gesättigter Pflanzenfettsäuren mittlerer Kettenlänge und mit einem Anteil einer essentiellen Fettsäure |
| 3,000 g | 2-(p-Dimethylaminophenyl)-benzoxazol-5-sulfonsäure |
| 1,460 g | Triäthanolamin |
| 1,000 g | Glycerin |
| 0,200 g | Dowicil®200 (9) N-(3-Chlorallyl)-hexaminiumchlorid |
| 0,400 g | Parfum |
| ad 100 g | Entmineralisiertes Wasser |

Beispiel 15

Sonnenschutzcrème (Öl/Wasser-Emulsion)

| | |
|---|---|
| 15,000 g | Lanette®N (2) Kolloiddisperses Gemisch aus 90 Teilen Cetylstearylalkohol und 10 Teilen Natriumcetylstearylsulfat |
| 5,000 g | Parsol®MCX (1) Äthylhexyl-paramethoxy-zimtsäureester |
| 5,000 g | Cetiol®V (2) Ölsäuredecylester |
| 5,000 g | Vaselin® weiß |
| 0,100 g | p-Hydroxy-benzoesäure-propylester |
| 1,000 g | Deltyl® extra (1) Isopropylmyrisat |
| 2,000 g | PCL flüssig (10) Gemisch alkylverzweigter Fettsäureester |
| 5,000 g | 2-(p-Dimethylaminophenyl)-benzoxazol-5-sulfonsäure |
| 0,600 g | Natriumhydroxyd |
| 4,000 g | Propylenglykol |
| 0,200 g | p-Hydroxybenzoesäure-methylester |
| 0,200 g | Germall®115 (4) Imidazolidinyl-harnstoffverbindung |
| 0,500 g | Parfum |
| ad 100 g | Entmineralisiertes Wasser |

Beispiel 16

Sonnenschutzgel

| | |
|---|---|
| 5,000 g | Parsol®MCX (1) Äthylhexyl-paramethoxy-zimtsäureester |
| 13,000 g | Emulgin®B (2) Cetylstearylalkohol mit ca. 30 Mol Äthylenoxyd |
| 20,000 g | Cetiol®HE (2) Polyol-Fettsäureester |
| 0,500 g | Phenonip® (7) Gemisch von p-Hydroxybenzoesäureestern in 2-Phenoxy-äthylalkohol |
| 2,000 g | Deltyl®extra (1) Isopropylmyristat |
| 5,000 g | 2-(p-Dimethylaminophenyl)-benzoxazol-5-sulfonsäure |
| 3,100 g | Triäthanolamin |
| 5,000 g | Propylenglykol |
| 0,500 g | Parfum |
| ad 100 g | Entmineralisiertes Wasser |

## Beispiel 17

### Haarwasser »A«, »B« und »C«

Haarwasser »A« (Kontrolle)

| | | |
|---|---|---|
| | 45,000 g | Feinsprit |
| | 0,100 g | Parfum |
| | 0,250 g | D-Panthenol-äthyläther |
| | 0,250 g | D-Penthenol |
| | 0,050 g | Cetylpyridiniumchlorid |
| | 0,600 mg | F-, D- + C-Blue No. 1, Col.Ind. No. 42 090 (Triphenylmethan-Farbstoff) |
| ad | 100 g | Entmineralisiertes Wasser |

Haarwasser »B«

Gleich wie »A« mit zusätzlich 0,025 g 2-(p-Dimethylaminophenyl)-benzoxazol-5-sulfonsäure und 0,013 g Triäthanolamin.

Haarwasser »C«

Gleich wie »A« mit zusätzlich 0,050 g 2-(p-Dimethylaminophenyl)-benzoxazol-5-sulfonsäure und 0,026 g Triäthanolamin.

### Lichttest (Xenotest)

Die obigen Mittel A, B und C werden einem Xenotest unterzogen. Die 3stündige bzw. 6stündige Belichtung entspricht einer ca. 3monatigen bzw. 6monatigen Belichtung im Labor bei Kunstlicht (1000 Lux) während 10–12 Stunden/Tag. Das Aussehen des Mittels nach der Belichtung wird jeweils mit dem eines entsprechenden unbelichteten Mittels verglichen. Die Ergebnisse sind die folgenden:

| Haarwasser | Aussehen nach 3stündiger Belichtung | Aussehen nach 6stündiger Belichtung |
|---|---|---|
| A | deutlicher Farbumschlag nach Grün, heller | starker Farbumschlag nach Türkis |
| B | unverändert | kein Farbumschlag, jedoch Spur heller |
| C | unverändert | kein Farbumschlag, jedoch Spur heller |

## Beispiel 18

### Haarwasser »D«, »E« und »F«

Haarwasser »D«

| | | |
|---|---|---|
| | 45,000 g | Feinsprit |
| | 0,100 g | Parfum |
| | 0,500 g | Isoadipat (Diisopropyladipat) |
| | 0,800 mg | F-, D- + C-Yellow No. 6, Col.Ind. No. 15 985 (Monoazo-Farbstoff) |
| ad | 100 g | Entmineralisiertes Wasser |

Haarwasser »E«

Gleich wie »D« mit zusätzlich 0,025 g 2-(p-Dimethylaminophenyl)-benzoxazol-5-sulfonsäure und 0,013 g Triäthanolamin.

Haarwasser »F«

Gleich wie »D« mit zusätzlich 0,050 g 2-(p-Dimethylaminophenyl)-benzoxazol-5-sulfonsäure und 0,026 g Triäthanolamin.

Lichttest (Xenotest)

Die obigen drei Haarwasser werden in analoger Weise gemäß Beispiel 17 einem Xenotest unterzogen. Die Ergebnisse sind wie folgt:

| Haarwasser | Aussehen nach 3stündiger Belichtung | Aussehen nach 6stündiger Belichtung |
|---|---|---|
| D | unverändert | leichter Farbumschlag, gelber und weniger orange |
| E | unverändert | unverändert |
| F | unverändert | unverändert |

| Haarwasser | Aussehen nach 12stündiger Belichtung | Aussehen nach 27stündiger Belichtung |
|---|---|---|
| D | leichter Farbumschlag, gelber und weniger orange | gelber und weniger orange |
| E | unverändert | unverändert |
| F | unverändert | unverändert |

Beispiel 19

Farbfestiger: Goldblond »A«, »B« und »C«

Goldblond »A«

| | |
|---|---|
| 45,000 g | Feinsprit |
| 5,000 g | Aristoflex®A 60% (11) Vinylacetat-Mischpolymerisat |
| 0,200 g | Citroflex®A-2 (12) Acetyltriäthylcitrat |
| 49,350 g | Entmineralisiertes Wasser |
| 0,320 g | Ammoniumhydroxyd 25% |
| 0,100 g | Parfum |
| 0,15 g | F-, D- + C-Yellow No. 6, Col.Ind. No. 15 985 (Monoazo-Farbstoff) |
| 0,015 g | F-, D- + C-Yellow No. 5, Col.Ind. No. 19 140 (Pyrazolon-Farbstoff) |

Goldblond »B«

Gleich wie »A« mit zusätzlich 0,025 g 2-(p-Dimethylaminophenyl)-benzoxazol-5-sulfonsäure.

Goldblond »C«

Gleich wie »A« mit zusätzlich 0,050 g 2-(p-Dimethylaminophenyl)-benzoxazol-5-sulfonsäure.

Lichttest (Xenotest)

Die obigen drei Farbfestiger werden in analoger Weise zum Beispiel 17 einem Xenotest unterzogen. Die Ergebnisse sind wie folgt:

| Farbfestiger Goldblond | Aussehen nach 12stündiger Belichtung | Aussehen nach 24stündiger Belichtung | Aussehen nach 48stündiger Belichtung |
|---|---|---|---|
| A | unverändert | unverändert | Spur heller und weniger orange |
| B | unverändert | unverändert | unverändert |
| C | unverändert | unverändert | unverändert |

Beispiel 20

Klares Shampoo »A«, »B«, »C« und »D«

Klares Shampoo »A«

| | | |
|---|---|---|
| | 25,000 g | Tego®-Betain L 7 (6) Fettsäureamidopropyldimethyl-aminoessigsäure-betain |
| | 20,000 g | Texapon®NT (2) Triäthanolaminlauryläthersulfat |
| | 3,000 g | Comperlan®KD (2) Kokosfettsäure-diäthanolamid |
| | 0,500 g | D-Panthenol-äthyläther |
| | 0,200 g | Dowicil®200 (9) N-(3-Chlorallyl)-hexaminiumhydrochlorid |
| | 1,500 g | F-, D- + C-Blue No. 1, Col.Ind. No. 42 090 (Triphenylmethan-Farbstoff) |
| ad | 100 g | Entmineralisiertes Wasser |

Klares Shampoo »B«

Gleich wie »A« mit zusätzlich 0,050 g 2-(p-Dimethylaminophenyl)-benzoxazol-5-sulfonsäure und 0,026 g Triäthanolamin.

Klares Shampoo »C«

Gleich wie »A« mit zusätzlich 0,050 g Uvinul®MS 40 (13) 2-Hydroxy-4-methoxy-benzophenon-5-sulfonsäure.

Klares Shampoo »D«

Gleich wie »A« mit zusätzlich 0,050 g Uvinul®D 50 (13) 2,2',4,4'-Tetrahydroxybenzophenon. Bemerkung: Unlöslich (1 Stunde Rühren bei 50° — Klumpenbildung).

Lichttest (Xenotest)

Die obigen klaren blauen Shampoos »A«, »B« und »C« werden in analoger Weise zum Beispiel 17 und unter gleichen Bedingungen einem Xenotest unterworfen. Die Ergebnisse sind wie folgt:

| Klares Shampoo | Aussehen nach 3stündiger Belichtung | Aussehen nach 6stündiger Belichtung |
|---|---|---|
| A | Rosa | Rosa |
| B | Blau, jedoch Spur heller | Hellblau |
| C | praktisch farblos | Rosa |

Beispiel 21

Nicht-Aerosol Haarspray

| | |
|---|---|
| 10,00 g | Gantrez®ES 425 (13) Poly(Methylvinyläther/Maleinsäuremonoalkylester) |
| 0,10 g | Diisopropyladipat |
| 0,10 g | Dow Corning 556 Fluid (14) Polyphenylmethylsiloxan |
| 84,00 g | Feinsprit |
| 0,20 g | Parfum |
| 1,20 g | Ammoniumhydroxid 25% |
| 0,10 g | Genamin®KDM (11) Alkyltrimethylammoniumchlorid, Alkyl = vorwiegend $C_{20}-C_{22}$ |
| 0,50 g | 2-(p-Dimethylaminophenyl)-benzoxazol-5-sulfonsäure |
| 3,80 g | Entmineralisiertes Wasser |
| 100,00 g | |

Die Hersteller der in den Beispielen 12 bis 21 angegebenen Ingredienzien sind:

1) Givaudan S.A.
2) Henkel + Cie GmbH
3) Goodrich Chemical Co.
4) Sutton Laboratories
5) BASF AG
6) Th. Goldschmidt AG
7) Nipa Laboratories
8) Dynamit Nobel AG
9) Dow Chemical Co.
10) Dragoco AG
11) Hoechst AG
12) Pfizer AG
13) GAF Corp.
14) Dow Corning Corp.

**Patentansprüche für die Vertragsstaaten BE, CH, DE, FR, GB, IT, NL, SE**

1. Verbindungen der allgemeinen Formel

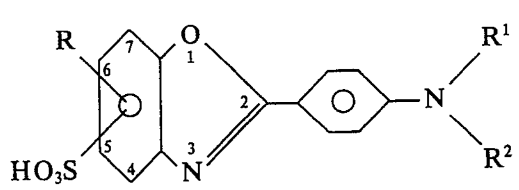

(I)

worin R Wasserstoff oder Alkyl mit 1 bis 4 Kohlenstoffatomen in 5- oder 6-Stellung, und $R^1$ und $R^2$ unabhängig voneinander Wasserstoff, Alkyl mit 1 bis 4 Kohlenstoffatomen oder eine Gruppe $-(XO)_n-H$ bedeuten, worin X eine gegebenenfalls durch Methyl oder Äthyl substituierte Äthylengruppe mit 2 bis 4 Kohlenstoffatomen und n eine ganze Zahl von 1 bis 10 bedeuten, wobei mindestens eines der Symbole $R^1$ und $R^2$ von Wasserstoff verschieden ist, oder $R^1$ und $R^2$ zusammen mit dem Stickstoffatom, an das sie geknüpft sind, einen 5- bis 7gliedrigen, gesättigten Ring bilden, der gegebenenfalls noch ein weiteres Heteroatom enthält, und Salze dieser Verbindungen.

2. Verbindungen der allgemeinen Formel

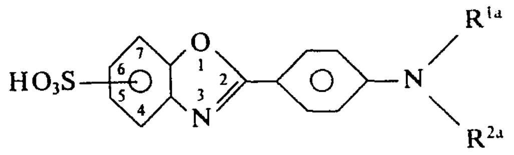

(Ia)

worin $R^{1a}$ und $R^{2a}$ unabhängig voneinander Wasserstoff, Alkyl mit 1 bis 4 Kohlenstoffatomen oder eine Gruppe $-XaOH$ bedeuten, worin Xa eine gegebenenfalls mit Methyl oder Äthyl substituierte Äthylengruppe mit 2 bis 4 Kohlenstoffatomen bedeutet, wobei mindestens eines der Symbole $R^{1a}$ und $R^{2a}$ von Wasserstoff verschieden ist oder $R^{1a}$ und $R^{2a}$ zusammen mit dem Stickstoffatom, an das sie geknüpft sind, einen 5- bis 7gliedrigen, gesättigten Ring bilden, der gegebenenfalls noch ein weiteres Heteroatom enthält,
und Salze dieser Verbindungen.

3. Verbindungen nach Anspruch 1, worin R Wasserstoff bedeutet.

4. Verbindungen nach einem der Ansprüche 1 bis 3, worin $R^1$ bzw. $R^{1a}$ Methyl bedeutet.

5. Verbindungen nach einem der Ansprüche 1 bis 4, worin $R^2$ bzw. $R^{2a}$ Methyl bedeutet.

6. Verbindungen nach einem der Ansprüche 1 bis 5, worin die Sulfonsäuregruppe in 5-Stellung ist.

7. 2-(p-Dimethylaminophenyl)-benzoxazol-5-sulfonsäure und deren Salze.

8. 2-(p-Methylaminophenyl)-benzoxazol-5-sulfonsäure.

9. 2-(p-Dimethylaminophenyl)-benzoxazol-6-sulfonsäure.

10. 2-(p-Dimethylaminophenyl)-5-methyl-benzoxazol-6-sulfonsäure.

11. 2-(p-Dimethylaminophenyl)-6-methyl-benzoxazol-5-sulfonsäure.

12. Verbindungen nach einem der Ansprüche 1 bis 11 als UV-A-Filter.

13. Verbindungen nach einem der Ansprüche 1 bis 11 als UV-A-Filter für Lichtschutzmittel, als UV-A-Filter zum Schutz von Farbstoffen in Farbstoffe enthaltenden kosmetischen Mitteln oder als UV-A-Filter zum Schutz der Haare in Haarpflegemitteln.

14. Verfahren zur Herstellung von Verbindungen der allgemeinen Formel I in Anspruch 1 und von Salzen dieser Verbindungen, dadurch gekennzeichnet, daß man

a) eine Verbindung der Formel

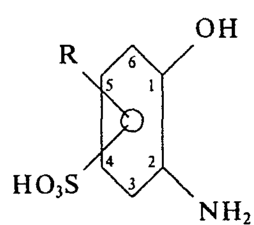

(II)

worin R Wasserstoff oder Alkyl mit 1 bis 4 Kohlenstoffatomen in 4- oder 5-Stellung bedeutet, mit einer Verbindung der Formel

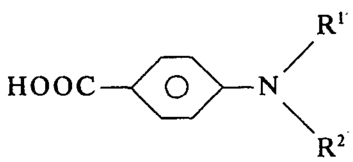

(III)

worin $R^{1'}$ Wasserstoff oder Alkyl mit 1 bis 4 Kohlenstoffatomen und $R^{2'}$ Alkyl mit 1 bis 4 Kohlenstoffatomen bedeuten oder $R^{1'}$ und $R^{2'}$ zusammen mit dem Stickstoffatom, an das sie geknüpft sind, einen 5- bis 7gliedrigen, gesättigten Ring bilden, der gegebenenfalls noch ein weiteres Heteroatom enthält,
oder einem reaktionsfähigen Derivat dieser Verbindung in Gegenwart eines Kondensationsmittels umsetzt, oder

b) eine Verbindung der Formel I, worin mindestens eines der Symbole $R^1$ und $R^2$ Wasserstoff bedeutet und das andere, wenn nicht Wasserstoff, Alkyl ist oder ein Salz einer solchen Verbindung mit einem Alkylenoxid oder einem Gemisch von Alkylenoxiden mit 2 bis 4 Kohlenstoffatomen umsetzt, oder

c) eine Verbindung der Formel

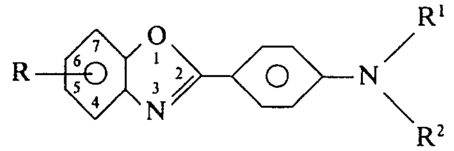

(IV)

worin R Wasserstoff oder Alkyl mit 1 bis 4 Kohlenstoffatomen in 5- oder 6-Stellung bedeutet und $R^1$ und $R^2$ die in Anspruch 1 angegebene Bedeutung besitzen, mit einem Sulfonierungsmittel behandelt,
und erwünschtenfalls eine erhaltene Verbindung der Formel I mit einer Base in ein Salz überführt.

15. Kosmetisches Mittel, dadurch gekennzeichnet, daß es als UV-A-Filter eine wirksame Menge von mindestens einer der Verbindungen der allgemeinen Formel I in Anspruch 1 oder von Salzen hiervon enthält.

16. Lichtschutzmittel, Farbstoff enthaltendes kosmetisches Mittel oder Haarpflegemittel, enthaltend mindestens eine der in den Ansprüchen 1 bis 11 genannten Verbindungen als UV-A-Filter

17. Verwendung von mindestens einer der in den Ansprüchen 1 bis 11 genannten Verbindungen als UV-A-Filter in einem Lichtschutzmittel, in einem Haarpflegemittel oder in einem Farbstoff enthaltenden kosmetischen Mittel.

**Patentansprüche für den Vertragsstaat AT**

1. Kosmetisches Mittel, dadurch gekennzeichnet, daß es als UV-A-Filter eine wirksame Menge von mindestens einer der Verbindungen der Formel

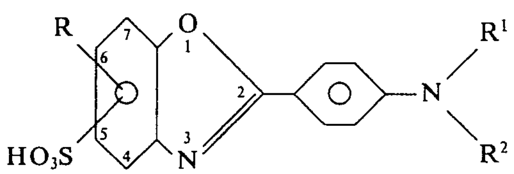

(I)

worin R Wasserstoff oder Alkyl mit 1 bis 4 Kohlenstoffatomen in 5- oder 6-Stellung und $R^1$ und $R^2$ unabhängig voneinander Wasserstoff, Alkyl mit 1 bis 4 Kohlenstoffatomen oder eine Gruppe $-(XO)_n-H$ bedeuten, worin X eine gegebenenfalls durch Methyl oder Äthyl substituierte Äthylengruppe mit 2 bis 4 Kohlenstoffatomen und n eine ganze Zahl von 1 bis 10 bedeuten, wobei mindestens eines der Symbole $R^1$ und $R^2$ von Wasserstoff verschieden ist oder $R^1$ und $R^2$ zusammen mit dem Stickstoffatom, an das sie geknüpft sind, einen 5- bis 7gliedrigen, gesättigten Ring bilden, der gegebenenfalls noch ein weiteres Heteroatom enthält,
oder von Salzen hiervon enthält.

2. Kosmetisches Mittel, dadurch gekennzeichnet, daß es als UV-A-Filter eine wirksame Menge von mindestens einer der Verbindungen der Formel

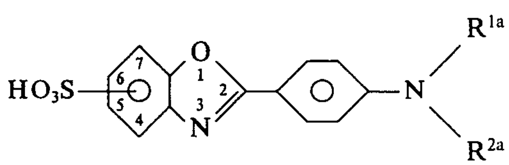

(Ia)

worin $R^{1a}$ und $R^{2a}$ unabhängig voneinander Wasserstoff, Alkyl mit 1 bis 4 Kohlenstoffatomen oder eine Gruppe $-XaOH$ bedeuten, worin Xa eine gegebenenfalls mit Methyl oder Äthyl substituierte Äthylengruppe mit 2 bis 4 Kohlenstoffatomen bedeutet, wobei mindestens eines der Symbole $R^{1a}$ und $R^{2a}$ von Wasserstoff verschieden ist, oder $R^{1a}$ und $R^{2a}$ zusammen mit dem Stickstoffatom, an das sie geknüpft sind, einen 5- bis 7gliedrigen, gesättigten Ring bilden, der gegebenenfalls noch ein weiteres Heteroatom enthält,
und Salze dieser Verbindungen.

3. Kosmetisches Mittel nach Anspruch 1, worin R in der Formel I Wasserstoff bedeutet.

4. Kosmetisches Mittel nach einem der Ansprüche 1 bis 3, worin $R^1$ bzw. $R^{1a}$ in der Formel I bzw. Ia Methyl bedeutet.

5. Kosmetisches Mittel nach einem der Ansprüche 1 bis 4, worin $R^2$ bzw. $R^{2a}$ in der Formel I bzw. Ia Methyl bedeutet.

6. Kosmetisches Mittel nach einem der Ansprüche 1 bis 5, worin die Sulfonsäuregruppe in der Formel I bzw. Ia in 5-Stellung ist.

7. Kosmetisches Mittel, dadurch gekennzeichnet, daß es als UV-A-Filter eine wirksame Menge von 2-(p-Dimethylaminophenyl)-benzoxazol-5-sulfonsäure und/oder einem Salz davon enthält.

8. Kosmetisches Mittel, dadurch gekennzeichnet, daß es als UV-A-Filter eine wirksame Menge von 2-(p-Methylaminophenyl)-benzoxazol-5-sulfonsäure enthält.

9. Kosmetisches Mittel, dadurch gekennzeichnet, daß es als UV-A-Filter eine wirksame Menge von 2-(p-Dimethylaminophenyl)-benzoxazol-6-sulfonsäure enthält.

10. Kosmetisches Mittel, dadurch gekennzeichnet, daß es als UV-A-Filter eine wirksame Menge von 2-(p-Dimethylaminophenyl)-5-methyl-benzoxazol-6-sulfonsäure enthält.

11. Kosmetisches Mittel, dadurch gekennzeichnet, daß es als UV-A-Filter eine wirksame Menge von 2-(p-Dimethylaminophenyl)-6-methyl-benzoxazol-5-sulfonsäure enthält.

12. Lichtschutzmittel, Farbstoff enthaltendes kosmetisches Mittel oder Haarpflegemittel, enthaltend mindestens eine der in den Ansprüchen 1 bis 11 genannten Verbindungen als UV-A-Filter.

13. Verfahren zur Herstellung von Verbindungen der allgemeinen Formel I in Anspruch 1 und von Salzen dieser Verbindungen, dadurch gekennzeichnet, daß man

a) eine Verbindung der Formel

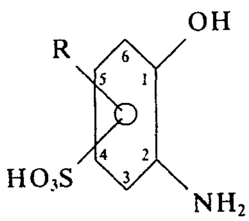

(II)

worin R Wasserstoff oder Alkyl mit 1 bis 4 Kohlenstoffatomen in 4- oder 5-Stellung bedeutet, mit einer Verbindung der Formel

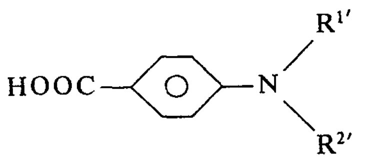

(III)

worin $R^{1'}$ Wasserstoff oder Alkyl mit 1 bis 4 Kohlenstoffatomen und $R^{2'}$ Alkyl mit 1 bis 4 Kohlenstoffatomen bedeuten oder $R^{1'}$ und $R^{2'}$ zusammen mit dem Stickstoffatom, an das sie geknüpft sind, einen 5- bis 7gliedrigen, gesättigten Ring bilden, der gegebenenfalls noch ein weiteres Heteroatom enthält,
oder einem reaktionsfähigen Derivat dieser Verbindung in Gegenwart eines Kondensationsmittels umsetzt, oder

b) eine Verbindung der Formel I, worin mindestens eines der Symbole $R^1$ und $R^2$ Wasserstoff bedeutet und das andere, wenn nicht Wasserstoff, Alkyl ist, oder ein Salz einer solchen Verbindung, mit einem Alkylenoxid oder einem Gemisch von Alkylenoxiden mit 2 bis 4 Kohlenstoffatomen umsetzt, oder

c) eine Verbindung der Formel

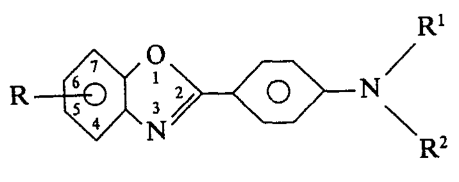

(IV)

worin R Wasserstoff oder Alkyl mit 1 bis 4 Kohlenstoffatomen in 5- oder 6-Stellung bedeutet und $R^1$ und $R^2$ die in Anspruch 1 angegebene Bedeutung besitzen,
mit einem Sulfonierungsmittel behandelt,
und erwünschtenfalls eine erhaltene Verbindung der Formel I mit einer Base in ein Salz überführt.

14. Verfahren zur Herstellung eines kosmetischen Mittels, dadurch gekennzeichnet, daß man mindestens eine der in den Ansprüchen 1 bis 11 genannten Verbindungen mit in der Kosmetik üblichen Wirkstoffen, Hilfsstoffen und Trägermaterialien auf in der Kosmetik übliche Weise vermischt.

15. Verwendung von mindestens einer der in den Ansprüchen 1 bis 11 genannten Verbindungen als UV-A-Filter in einem Lichtschutzmittel, in einem Haarpflegemittel oder in einem Farbstoff enthaltenden kosmetischen Mittel.

**Claims for the contracting states: BE, CH, DE, FR, GB, IT, NL, SE**

1. Compounds of the general formula

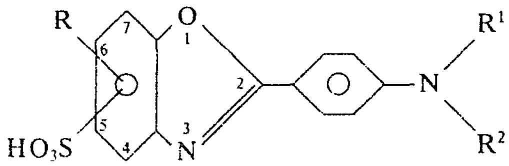

(I)

wherein R signifies hydrogen or alkyl with 1 to 4 carbon atoms in the 5- or 6-position, and $R^1$ and $R^2$ independently of one another signify hydrogen, alkyl with 1 to 4 carbon atoms or a group $-(XO)_n-H$, wherein X signifies an ehtylene group which is optionally substituted by methyl or ethyl with 2 to 4 carbon atoms, and n signifies a whole number of 1 to 10, at least one of the symbols $R^1$ and $R^2$ being different from hydrogen, or $R^1$ and $R^2$ together with the nitrogen atom to which they are attached form a 5- to 7-membered saturated ring which optionally contains a further hetero atom,
and salts of these compounds.

2. Compounds of the general formula

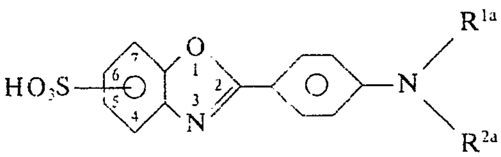

(Ia)

wherein $R^{1a}$ and $R^{2a}$ independently of one another signify hydrogen, alkyl with 1 to 4 carbon atoms or a group $-XaOH$, wherein Xa signifies an ethylene group which is optionally substituted with methyl or ethyl with 2 to 4 carbon atoms, at least one of the symbols $R^{1a}$ and $R^{2a}$ being different from hydrogen, or $R^{1a}$ and $R^{2a}$ together with the nitrogen atom to which they are attached form a 5- to 7-membered saturated ring which optionally contains a further hetero atom,
and salt of these compounds.

3. Compounds according to claim 1, wherein R signifies hydrogen.
4. Compounds according to one of claims 1 to 3, wherein $R^1$ or $R^{1a}$ signifies methyl.
5. Compounds according to one of claims 1 to 4, wherein $R^2$ or $R^{2a}$ signifies methyl.
6. Compounds according to one of claims 1 to 5, wherein the sulphonic acid group is in the 5-position.
7. 2-(p-Dimethylaminophenyl)-benzoxazole-5-sulphonic acid and its salts.
8. 2-(p-Methylaminophenyl)-benzoxazole-5-sulphonic acid.
9. 2-(p-Dimethylaminophenyl)-benzoxazole-6-sulphonic acid.
10. 2-(p-Dimethylaminophenyl)-5-methyl-benzoxazole-6-sulphonic acid.
11. 2-(p-Dimethylaminophenyl)-6-methyl-benzoxazole-5-sulphonic acid.
12. Compounds according to one of claims 1 to 11 as UV-A filters.
13. Compounds according to one of claims 1 to 11 as UV-A filters for light-screen compositions, as UV-A filters for the protection of colouring substances in cosmetic compositions containing colouring substances or as UV-A filters for the protection of the hair in haircare compositions.
14. Process for the manufacture of compounds of general formula I in claim 1 and of salts of these compounds, characterized by

a) reacting a compound of the formula

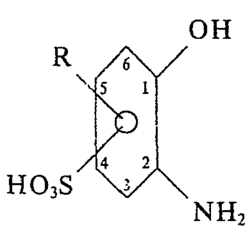

(II)

wherein R signifies hydrogen or alkyl with 1 to 4 carbon atoms in the 4- or 5-position, with a compound of the formula

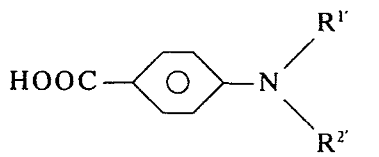

(III)

wherein $R^{1'}$ signifies hydrogen or alkyl with 1 to 4 carbon atoms, and $R^{2'}$ signifies alkyl with 1 to 4 carbon atoms, or $R^{1'}$ and $R^{2'}$ together with the nitrogen atom to which they are attached form a 5- to 7-membered saturated ring which optionally contains a further hetero atom,
or a reactive derivative of this compound, in the presence of a condensation agent, or

b) reacting a compound of formula I wherein at least one of the symbols $R^1$ and $R^2$ signifies hydrogen and the other, when not hydrogen, is alkyl, or a salt of such a compound, with an alkylene oxide or a mixture of alkylene oxides with 2 to 4 carbon atoms, or

c) treating a compound of the formula

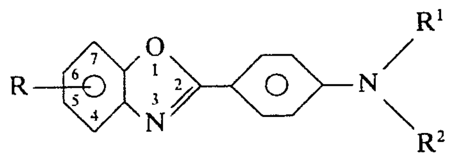

(IV)

wherein R signifies hydrogen or alkyl with 1 to 4 carbon atoms in the 5- or 6-position, and $R^1$ and $R^2$ have the significance given in claim 1,
with a sulphonating agent,
and, if desired, converting a compound of formula I obtained with a base into a salt.

15. Cosmetic composition, characterized in that it contains as a UV-A filter an effective amount of at least one of the compounds of general formula I in claim 1 or of salts thereof.

16. Light-screen composition, cosmetic composition containing colouring substance or hair-care composition containing at least one of the compounds set forth in claims 1 to 11 as a UV-A filter.

17. Use of at least one of the compounds set forth in claims 1 to 11 as a UV-A filter in a light-screen composition, in a hair-care composition or in a cosmetic composition containing colouring substance.

**Claims for the contracting state: AT**

1. Cosmetic composition, characterized in that it contains as a UV-A filter an effective amount of at least one of the compounds of the formula

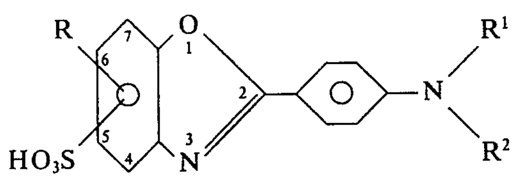

(I)

wherein R signifies hydrogen or alkyl with 1 to 4 carbon atoms in the 5- or 6-position, and $R^1$ and $R^2$ independently of one another signify hydrogen, alkyl with 1 to 4 carbon atoms or a group $-(XO)_n-H$, wherein X signifies an ethylene group which is optionally substituted by methyl or ethyl with 2 to 4 carbon atoms, and n signifies a whole number of 1 to 10, at least one of the symbols $R^1$ and $R^2$ being different from hydrogen, or $R^1$ and $R^2$ together with the nitrogen atom to which they are attached form a 5- to 7-membered saturated ring which optionally contains a further hetero atom,
or of salts thereof.

2. Cosmetic composition, characterized in that it contains as a UV-A filter an effective amount of at least one of the compounds of the formula

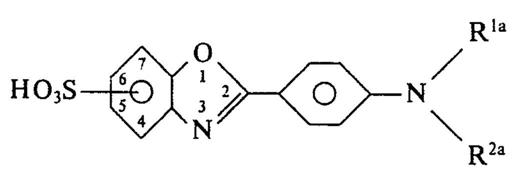

(Ia)

wherein $R^{1a}$ and $R^{2a}$ independently of one another signify hydrogen, alkyl with 1 to 4 carbon atoms or a

group $-XaOH$, wherein Xa signifies an ethylene group which is optionally substituted with methyl or ethyl with 2 to 4 carbon atoms, at least one of the symbols $R^{1a}$ and $R^{2a}$ being different from hydrogen, or $R^{1a}$ and $R^{2a}$ together with the nitrogen atom to which they are attached form a 5- to 7-membered saturated ring which optionally contains a further hetero atom,
and salts of these compounds.

3. Cosmetic composition according to claim 1, wherein R in formula I signifies hydrogen.

4. Cosmetic composition according to one of claims 1 to 3, wherein $R^1$ or $R^{1a}$ in formula I or Ia signifies methyl.

5. Cosmetic composition according to one of claims 1 to 4, wherein $R^2$ or $R^{2a}$ in formula I or Ia signifies methyl.

6. Cosmetic composition according to one of claims 1 to 5, wherein the sulphonic acid group in formula I or Ia is in the 5-position.

7. Cosmetic composition, characterized in that it contains as a UV-A filter an effective amount of 2-(p-dimethylaminophenyl)-benzoxazole-5-sulphonic acid and/or a salt thereof.

8. Cosmetic composition, characterized in that it contains as a UV-A filter an effective amount of 2-(p-methylaminophenyl)-benzoxazole-5-sulphonic acid.

9. Cosmetic composition, characterized in that it contains as a UV-A filter an effective amount of 2-(p-dimethylaminophenyl)-benzoxazole-6-sulphonic acid.

10. Cosmetic composition, characterized in that it contains as a UV-A filter an effective amount of 2-(p-dimethylaminophenyl)-5-methyl-benzoxazole-6-sulphonic acid.

11. Cosmetic composition, characterized in that it contains as a UV-A filter an effective amount of 2-(p-dimethylaminophenyl)-6-methyl-benzoxazole-5-sulphonic acid.

12. Light-screen composition, cosmetic composition containing colouring substance or hair-care composition containing at least one of the compounds set forth in claims 1 to 11 as a UV-A filter.

13. Process for the manufacture of compounds of general formula I in claim 1 and of salts of these compounds, characterized by

a) reacting a compound of the formula

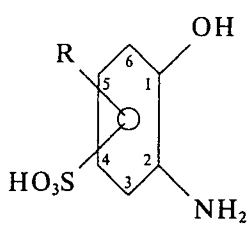

(II)

wherein R signifies hydrogen or alkyl with 1 to 4 carbon atoms in the 4- or 5-position, with a compound of the formula

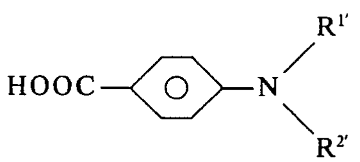

(III)

wherein $R^{1'}$ signifies hydrogen or alkyl with 1 to 4 carbon atoms, and $R^{2'}$ signifies alkyl with 1 to 4 carbon atoms, or $R^{1'}$ and $R^{2'}$ together with the nitrogen atom to which they are attached form a 5- to 7-membered saturated ring which optionally contains a further hetero atom,
or a reactive derivative of this compound in the presence of a condensation agent, or

b) reacting a compound of formula I wherein at least one of the symbols $R^1$ and $R^2$ signifies hydrogen and the other, when not hydrogen, is alkyl, or a salt of such a compound, with an alkylene oxide or a mixture of alkylene oxides with 2 to 4 carbon atoms, or

c) treating a compound of the formula

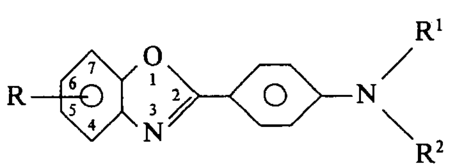

(IV)

wherein R signifies hydrogen or alkyl with 1 to 4 carbon atoms in the 5- or 6-position, and $R^1$ and $R^2$ have the significance given in claim 1,

with a sulphonating agent,
and, if desired, converting a compound of formula I obtained with a base into a salt.

14. Process for the manufacture of a cosmetic composition, characterized by mixing in a manner which is usual in cosmetics at least one of the compounds set forth in claims 1 to 11 with active substances, adjuvant substances and carrier materials which are usual in cosmetics.

15. Use of at least one of the compounds set forth in claims 1 to 11 as a UV-A filter in a light-screen composition, in a hair-care composition or in a cosmetic composition containing colouring substance.

**Revendications pour les états contractants: BE, CH, DE, FR, GB, IT, NL, SE**

1. Composés de formule générale

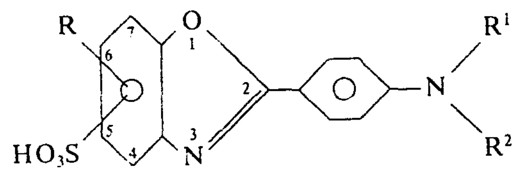

(I)

où R représente un hydrogène ou un alcoyle en $C_1$ à $C_4$ en position 5 ou 6, et $R^1$ et $R^2$ représente indépendamment l'un de l'autre un hydrogène, un alcoyle en $C_1$ à $C_4$ ou un groupe $-(XO)_n-H$, où X représente un groupe éthylène éventuellement substitué par un méthyle ou un éthyle en $C_2$ à $C_4$ et n un nombre entier allant de 1 à 10, où au moins l'un des symboles $R^1$ et $R^2$ est différent d'un hydrogène, ou bien où $R^1$ et $R^2$ forment avec l'atome d'azote auquel ils sont liés un noyau saturé à 5 à 7 chaînons, qui peut encore éventuellement contenir un autre hétéroatome,
et les sels de ces composés.

2. Composés de formule générale

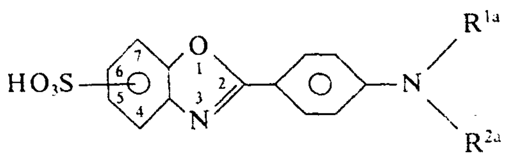

(Ia)

où $R^{1a}$ et $R^{2a}$ représentent indépendamment l'un de l'autre un hydrogène, un alcoyle en $C_1$ à $C_4$ ou un groupe $-XaOH$, où Xa représente un groupe éthylène éventuellement substitué par un méthyle ou un éthyle en $C_2$ à $C_4$, où au moins l'un des symboles $R^{1a}$ et $R^{2a}$ est différent d'un hydrogène, ou bien où $R^{1a}$ et $R^{2a}$ forment ensemble avec l'atome d'azote auquel ils sont liés un noyau saturé à 5 à 7 chaînons, qui contient éventuellement encore un autre hétéroatome,
et les sels de ces composés.

3. Composés selon la revendication 1, où R représente un hydrogène.

4. Composés selon l'une des revendications 1 à 3, où $R^1$ ou $R^{1a}$ représente un méthyle.

5. Composés selon l'une des revendications 1 à 4, où $R^2$ ou $R^{2a}$ représente un méthyle.

6. Composés selon l'une des revendications 1 à 5, où le groupe acide sulfonique est en position 5.

7. L'acide 2-(p-diméthylaminophényl)benzoxazol-5-sulfonique et ses sels.

8. L'acide 2-(p-méthylaminophényl)-benzoxazol-5-sulfonique.

9. L'acide 2-(p-diméthylaminophényl)-benzoxazol-6-sulfonique.

10. L'acide 2-(p-diméthylaminophényl)-5-méthy enzoxazol-6-sulfonique.

11. L'acide 2-(p-diméthylaminophényl)-6-méthyl-benzoxazol-5-sulfonique.

12. Composés selon l'une des revendications 1 à 11 comme filtres UV-A.

13. Composés selon l'une des revendications 1 à 11 comme filtres UV-A pour écrans solaires, comme filtres UV-A pour protéger les colorants dans les agents cosmétiques contenant des colorants ou comme filtres UV-A pour protéger les cheveux dans les agents de traitement des cheveux.

14. Procédé de préparation de composés de formule générale I de la revendication 1 et de sels de ces composés, caractérisé en ce que

a) on fait réagir un composé de formule

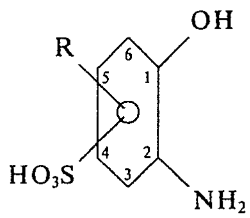

(II)

où R représente un hydrogène ou un alcoyle en $C_1$ à $C_4$ en position 4 ou 5, avec un composé de formule

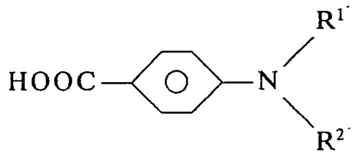

(III)

où $R^{1'}$ représente un hydrogène ou un alcoyle en $C_1$ à $C_4$, et $R^{2'}$ un alcoyle en $C_1$ à $C_4$, ou bien où $R^{1'}$ et $R^{2'}$ forment ensemble avec l'atome d'azote auxquels ils sont liés un noyau saturé à 5 à 7 chaînons, qui contient éventuellement encore un autre hétéroatome,
ou avec un dérivé réactif de ce composé,
en présence d'un agent de condensation, ou

b) on fait réagir un composé de formule I, où au moins l'un des symboles $R^1$ et $R^2$ représente un hydrogène et l'autre, lorsqu'il n'est pas un hydrogène, est un alcoyle, ou un sel d'un tel composé, avec un alcoylènoxyde ou un mélange d'alcoylènoxydes en $C_2$ à $C_4$, ou

c) on traite un composé de formule

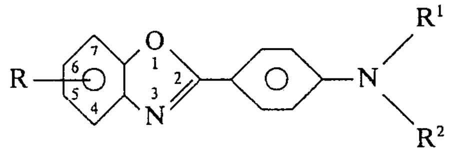

(IV)

où R représente un hydrogène ou un alcoyle en $C_1$ à $C_4$ en position 5 ou 6, et $R^1$ et $R^2$ ont la signification donnée dans la revendication 1,
avec un agent de sulfonation,
et si on le désire on transforme un composé de formule I obtenu avec une base en un sel.

15. Agent cosmétique, caractérisé en ce qu'il contient comme filtre UV-A une quantité efficace d'au moins un des composés de formule générale I de la revendication 1 ou de leurs sels.

16. Ecran solaire, agent cosmétique contenant un colorant ou agent de traitement des cheveux, contenant au moins l'un des composés mentionnés dans les revendications 1 à 11 comme filtre UV-A.

17. Application d'au moins un des composés mentionnés dans les revendications 1 à 11 comme filtre UV-A dans un écran solaire, dans un agent de traitement des cheveux ou dans un agent cosmétique contenant un colorant.

**Revendications pour l'état contractant: AT**

1. Agent cosmétique caractérisé en ce qu'il contient comme filtre UV-A une quantité efficace d'au moins un des composés de formule

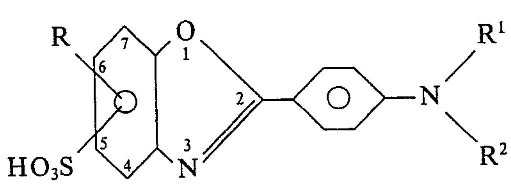

(I)

où R représente un hydrogène ou un alcoyle en $C_1$ à $C_4$ en position 5 ou 6, et $R^1$ et $R^2$ représentent indépendamment l'un de l'autre un hydrogène, un alcoyle en $C_1$ à $C_4$ ou un groupe $-(XO)_n-H$, où X représente un groupe éthylène éventuellement substitué par un méthyle ou un éthyle, et ayant de 2 à 4 atomes de carbone, et n un nombre entier allant de 1 à 10, où au moins l'un des symboles $R^1$ et $R^2$ est différent d'un hydrogène, ou bien où $R^1$ et $R^2$ forment ensemble avec l'atome d'azote auquel ils sont

liés un noyau saturé à 5 à 7 chaînons qui contient eventuellement encore un autre héteroatome, ou de ses sels.

2. Agent cosmétique caractérisé en ce qu'il contient comme filtre UV-A une quantité efficace d'au moins un des composés de formule

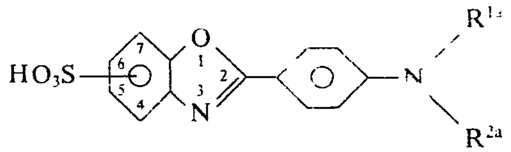

(Ia)

où $R^{1a}$ et $R^{2a}$ representent indépendamment l'un de l'autre un hydrogene, un alcoyle en $C_1$ à $C_4$ ou un groupe $-XaOH$, où Xa représente un groupe éthylène éventuellement substitué par un méthyle ou un éthyle en $C_2$ à $C_4$, où au moins l'un des symboles $R^{1a}$ et $R^{2a}$ est différent d'un hydrogène, ou bien où $R^{1a}$ et $R^{2a}$ forment ensemble avec l'atome d'azote auquel ils sont liés un noyau saturé à 5 à 7 chaînons qui contient eventuellement encore un autre hétéroatome, et des sels de ces composés.

3. Agent cosmétique selon la revendication 1, où R dans la formule I représente un hydrogène.

4. Agent cosmétique selon l'une des revendications 1 à 3, où $R^1$ ou $R^{1a}$ dans la formule I ou Ia représente un méthyle.

5. Agent cosmétique selon l'une des revendications 1 à 4, où $R^2$ ou $R^{2a}$ dans la formule I ou Ia représente un méthyle.

6. Agent cosmétique selon l'une des revendications 1 à 5, où le groupe acide sulfonique dans la formule I ou Ia est en position 5.

7. Agent cosmétique caractérisé en ce qu'il contient comme filtre UV-A une quantité efficace d'acide 2-(p-diméthylaminophényl)-benzoxazol-5-sulfonique et/ou d'un de ses sels.

8. Agent cosmétique caractérisé en ce qu'il contient comme filtre UV-A une quantité efficace d'acide 2-(p-méthylaminophényl)-benzoxazol-5-sulfonique.

9. Agent cosmétique caractérisé en ce qu'il contient comme filtre UV-A une quantité efficace d'acide 2-(p-diméthylaminophényl)-benzoxazol-6-sulfonique.

10. Agent cosmétique caractérisé en ce qu'il contient comme filtre UV-A une quantité efficace d'acide 2-(p-diméthylaminophényl)-5-méthyl-benzoxazol-6-sulfonique.

11. Agent cosmétique caractérisé en ce qu'il contient comme filtre UV-A une quantité efficace d'acide 2-(p-diméthylaminophényl)-6-méthyl-benzoxazol-5-sulfonique.

12. Ecran solaire, agent cosmétique contenant un colorant ou agent de traitement des cheveux, contenant au moins un des composés mentionnés dans les revendications 1 à 11 comme filtre UV-A.

13. Procédé de preparation de composés de formule générale I de la revendication 1 et de sels de ces composés, caractérisé en ce que

a) on fait réagir un composé de formule

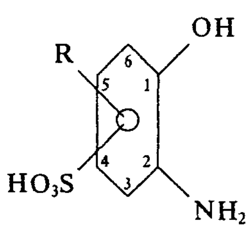

(II)

où R représente un hydrogène ou un alcoyle en $C_1$ à $C_4$ en position 4 ou 5, avec un composé de formule

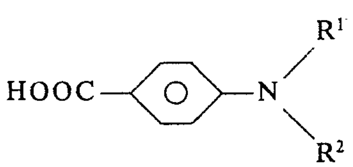

(III)

où $R^{1'}$ représente un hydrogène ou un alcoyle en $C_1$ à $C_4$, et $R^{2'}$ un alcoyle en $C_1$ à $C_4$, ou bien où $R^{1'}$ et $R^{2'}$ forment ensemble avec l'atome d'azote auquel ils sont liés un noyau saturé à 5 à 7 chaînons, qui contient éventuellement encore un autre hétéroatome, ou avec un dérivé réactif de ce composé, en présence d'un agent de condensation, ou

b) on fait réagir un composé de formule I, où au moins l'un des symboles $R^1$ et $R^2$ représente un hydrogène et l'autre, lorsqu'il n'est pas un hydrogène, est un alcoyle, ou un sel d'un tel composé,

avec un alcoylènoxyde ou un mélange d'alcoylènoxydes en $C_2$ à $C_4$, ou

c) on traite un composé de formule

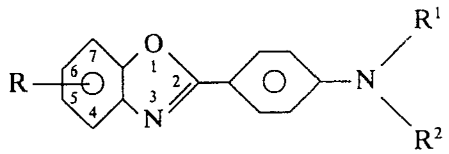

(IV)

où R représente un hydrogène ou un alcoyle en $C_1$ à $C_4$ en position 5 ou 6, et où $R^1$ et $R^2$ ont la signification donnée dans la revendication 1,

avec un agent sulfonant,

et si on le désire on transforme un composé de formule I obtenu avec une base en un sel.

14. Procédé de préparation d'un agent cosmétique, caractérisé en ce qu'on mélange au moins un des composés mentionnés dans les revendications 1 à 11 avec des matières actives, des adjuvants et des supports habituels en cosmétique, de manière habituelle en cosmétique.

15. Application d'au moins un des composés mentionnés dans les revendications 1 à 11 comme filtre UV-A dans un écran solaire, dans un agent de traitement des cheveux ou dans un agent cosmétique contenant un colorant.